# FASCICULE DE BREVET EUROPEEN

(11) **EP 0 429 344 B1**
(45) Date de publication et mention de la délivrance du brevet: **18.09.1996**
(21) Numéro de dépôt: 90403239.8
(22) Date de dépôt: 16.11.1990
(51) Int. Cl.: C07D 237/20, C07D 401/04, C07D 409/04, A61K 31/50

(54) **Dérivés de la pyridazine, procédé de préparation et compositions pharmaceutiques en contenant**
Pyridazin-Derivate, Verfahren zu ihrer Herstellung und diese enthaltende pharmazeutische Zusammensetzungen
Pyridazine derivatives, process for their preparation and pharmaceutical compositions containing them

(30) Priorité: 17.11.1989 FR 8915137; 15.06.1990 FR 9007533
(43) Date de publication de la demande: 29.05.1991
(73) Titulaire: SANOFI, F-75008 Paris (FR)
(72) Inventeur: Boigegrain, Robert, F-34830 Clapiers (FR); Brodin, Roger, F-34070 Montpellier (FR); Kan, Jean-Paul, F-34830 Clapiers (FR); Olliero, Dominique, F-34100 Montpellier (FR); Wermuth, Camille Georges, F-67100 Strasbourg (FR)
(74) Mandataire: Gillard, Marie-Louise

(56) Documents cités:
- EP-A- 382 634
- EP-A- 382 635
- EP-A- 0 072 726
- EP-A- 0 074 863

## Description

Depuis de nombreuses années, des dérivés de pyridazine ont été proposés en tant que médicaments, notamment actifs sur le système cardiovasculaire ou sur le système nerveux central.

En particulier, le brevet français 2 510 998 et le brevet européen 72 726 divulguent des dérivés de pyridazine diversement substitués sur le cycle pyridazinique et portant tous en position 3 un substituant aminé de du type 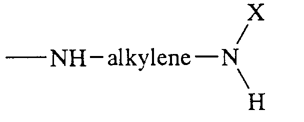 où X représente l'hydrogène ou un groupe (C₁-C₄)alkyle.

Le brevet européen 74 863 décrit des dérivés de pyridazine diversement substitués sur le cycle pyridazinique et portant tous en position 3 dudit cycle un susbtituant aminé du type 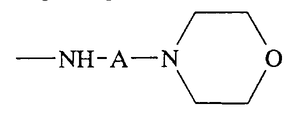 où A représente un alkylene en C₂-C₅.

Tous ces composés présentent une activité sur le système nerveux central en tant qu'antidépresseurs.

Selon la présente invention, on a maintenant trouvé des nouveaux dérivés de la pyridazine ayant perdu leur activité antidépressive et ayant acquis une activité intéressante en tant que ligands des récepteurs cholinergiques, en particulier les récepteurs de type M₁.

Selon un premier aspect, la présente invention a pour objet de nouveaux dérivés de pyridazine, choisis parmi :
* la 3-(2-diéthylamino-2-méthylpropyl)amino-6-phényl-5-propylpyridazine ;
* la 3-(2-diéthylamino-2-méthylpropyl)amino-6-(2-chlorophényl)-5-propylpyridazine ;
* la 3-(2-diéthylamino-2-méthylpropyl)amino-6-(2-méthoxyphényl)-5-méthylpyridazine ;
* la 3-(2-diéthylamino-2-méthylpropyl)amino-6-(2-hydroxyphényl)-5-méthylpyridazine ;
* la 3-(2-pipéridino-2-méthylpropyl)amino-6-(4-chlorophényl)-5-méthylpyridazine ;
* la 3-(2-pipéridino-2-méthylpropyl)amino-6-phényl-5-propylpyridazine ;
* la 3-(2-diéthylamino-2-méthylpropyl)amino-6-(4-chlorophényl)-5-méthylpyridazine ;
* la 3-(2-diéthylamino-2-méthylpropyl)amino-6-phényl-5-méthylpyridazine ;
* la 3-(2-diéthylamino-2-méthylpropyl)amino-5,6-diphénylpyridazine ;
* la 3-(2-diéthylamino-2-méthylpropyl)amino-6-(4-chlorophényl)-5-propylpyridazine ;
* la 3-(2-diéthylamino-2-méthylpropyl)amino-6-(4-méthoxyphényl)-5-propylpyridazine ;
* la 3-(2-diéthylamino-2-méthylpropyl)amino-6-(4-hydroxyphényl)-5-propylpyridazine ;
* la 3-(2-diéthylamino-2-méthylpropyl)amino-5-cyclopropyl-6-phénylpyridazine ;
* la 3-(2-diéthylamino-2-méthylpropyl)amino-5-isopropyl-6-(4-fluorophényl)pyridazine ;
* la 3-(2-(morpholin-4-yl)-2-méthylpropyl)amino-6-phényl-5-propylpyridazine ;
et leurs sels avec des acides minéraux ou organiques.

Les composés suivants sont particulièrement préférés :
- la 3-(2-diéthylamino-2-méthylpropyl)amino-6-phényl-5-propyl-pyridazine et ses sels ;
- la 3-(2-diéthylamino-2-méthylpropyl)amino-5,6-diphénylpyridazine et ses sels.

Les sels des composés selon la présente invention comprennent aussi bien ceux avec des acides minéraux ou organiques qui permettent une séparation ou une cristallisation convenable desdits composés, tels que l'acide picrique ou l'acide oxalique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate.

Selon un second aspect, la présente invention concerne un procédé de préparation des composés ci-dessus.

Selon la présente invention, le procédé de préparation des composés ci-dessus est caractérisé en ce que l'on fait réagir une amine de formule R₄NH₂ dans laquelle R₄ représente un groupe 2-diéthylamino-2-méthylpropyle ou le cas échéant 2-pipéridino-2-méthylpropyle ou 2-(morpholin-4-yl)-2-méthylpropyle, avec une chloro-6-pyridazine de formule : 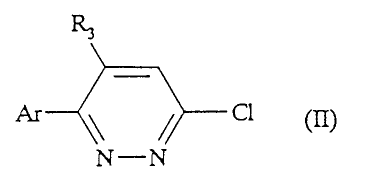 dans laquelle :
* R₃ représente un propyle et Ar représente un phényle ou le cas échéant un 2-chlorophényle, un 4-chlorophényle, un 4-méthoxyphényle ou un 4-hydroxyphényle ;
   ou
* R₃ représente un méthyle et Ar représente un 2-méthoxyphényle ou le cas échéant un 2-hydroxyphényle, un 4-chlorophényle ou un phényle ;
   ou
* R₃ représente un phényle et Ar représente un phényle ;
   ou
* R₃ représente un cyclopropyle et Ar un phényle ;
   ou
* R₃ représente un isopropyle et Ar un 4-fluorophényle ;
   et eventuellement on salifie le composé ainsi obtenu avec un acide minéral ou organique.

La réaction de substitution de la chloro-6 pyridazine (II) par l'amine R₄NH₂ est effectuée entre 100° et 150°C, éventuellement en présence de chlorure d'ammonium. On opère sans solvant, ou en présence d'un solvant inerte comme le n-butanol. Le dérivé pyridazinique selon l'invention est isolé par extraction et purifié, par exemple, par chromatographie.

Le produit ainsi obtenu est isolé, sous forme de base libre ou de sel, selon les techniques conventionnelles.

Lorsque ledit produit est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2 sulfonate.

A la fin de la réaction, le composé selon l'invention peut être isolé sous forme d'un de ses sels, par exemple le chlorhydrate ; dans ce cas, s'il est nécessaire, la base libre peut être préparée par neutralisation dudit sel avec une base minérale ou organique telle que l'hydroxyde de sodium ou la triéthylamine ou avec un carbonate ou bicarbonate alcalin, tel que le carbonate ou bicarbonate de sodium ou de potassium.

Lorsque Ar représente un hydroxyphényle, le composé selon l'invention est obtenu à partir du composé dans lequel Ar représente un alcoxyphényle et tous les autres substituants ont les définitions ci-dessus, par déalkylation en utilisant des méthodes connues.

Les chloro-6 pyridazines (II), utilisées comme produits de départ, sont préparées à partir des 2H-pyridazinones-3 (III) correspondantes par action d'un excès d'oxychlorure de phosphore à chaud, en opérant sans solvant ou en présence d'un solvant inerte tel que l'acétonitrile, selon le schéma réactionnel suivant :

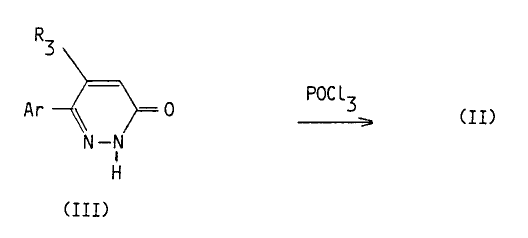

Les 2H-pyridazinones-3 (III) sont connues ou préparées par des méthodes connues.

Ainsi, lorsque R₃ est un phényle Ar', les 2H-pyridazinones-3 sont obtenues selon la méthode décrite par P. SCHMIDT et al. dans Helv. Chim. Acta, 1954, 15, 134-140, à partir du diéthylester de l'acide malonique et d'un dérivé d'hydrazone selon le schéma réactionnel suivant :

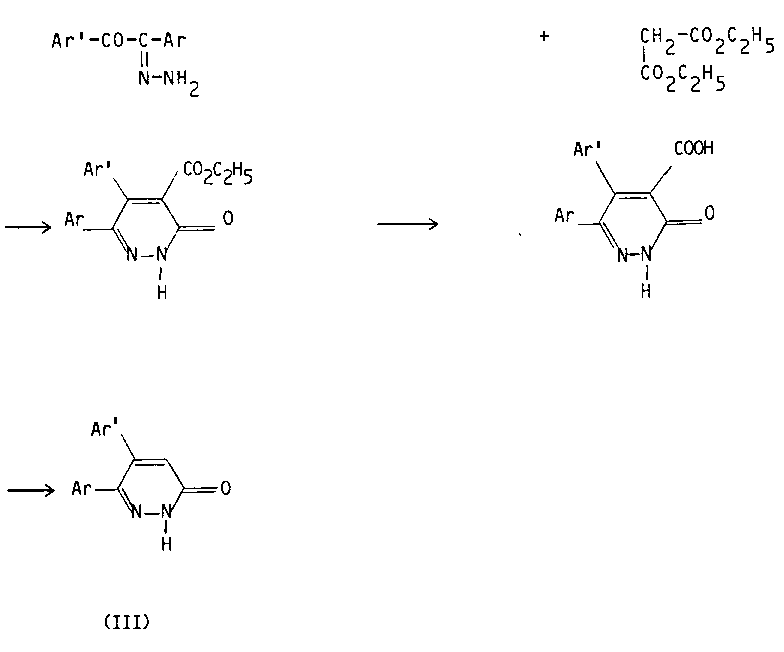

Lorsque R₃ représente un radical méthyle, propyle, isopropyle ou cyclopropyle, les composés (III) sont préparés à partir d'une cétone Ar-CO-CH₂R₃, (1) :

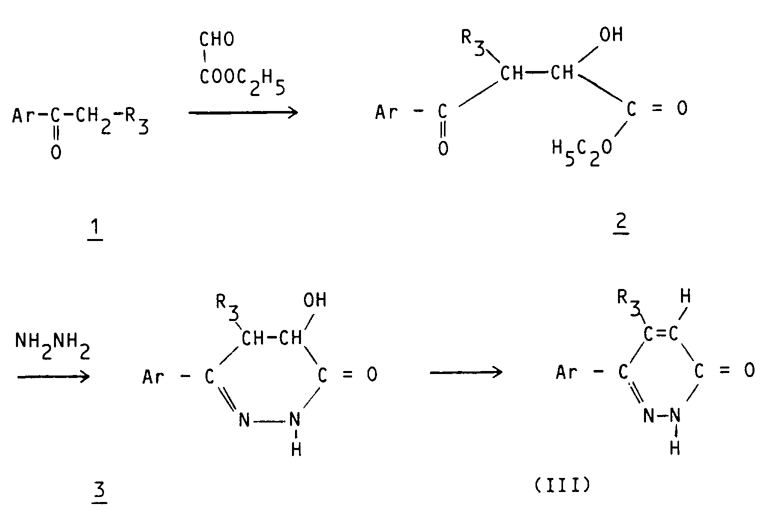

A partir de la cétone 1, par chauffage avec le glyoxylate d'éthyle à une température comprise entre 80 et 140°C, on obtient l'hydroxy cétoester 2. Le mélange réactionnel brut est alors repris dans un solvant inerte tel que le n-butanol et additionné d'hydrate d'hydrazine. Par chauffage au reflux pendant 24 heures, on obtient la dihydro-4,5 hydroxy-4 pyridazinone-3 3 qui, chauffée en milieu acide, conduit par déshydratation à la 2H-pyridazinone-3 (III).

Les amines R₄NH₂ sont connues ou préparées par des méthodes connues. Ainsi, elles peuvent être préparées à partir d'un dérivé cyané de formule :

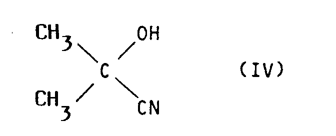

Par action d'une amine HNR₅R₆ (diéthylamine, pipéridine ou morpholine) en chauffant à une température comprise entre 40 et 80°C, éventuellement en présence d'un sel d'acide fort tel que le sulfate de sodium ou le sulfate de magnésium, on prépare d'abord un composé de formule : 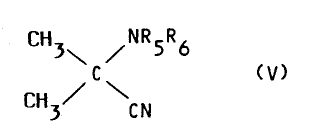 puis ce composé est hydraté par action d'un acide fort tel que l'acide sulfurique à chaud pour obtenir l'amide correspondant :

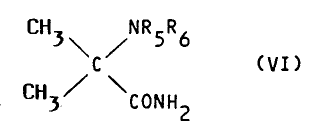

Enfin la réduction à chaud par un hydrure métallique tel qu'un hydrure de bore, ou un hydrure de lithium et d'aluminium permet d'obtenir l'amine R₄NH₂.

On peut également préparer une amine R₄NH₂ à partir d'un dérivé chloronitroso (VII) selon le mode opératoire décrit dans J. Prakt. Chem., 1978, 320 (3), 433-451.

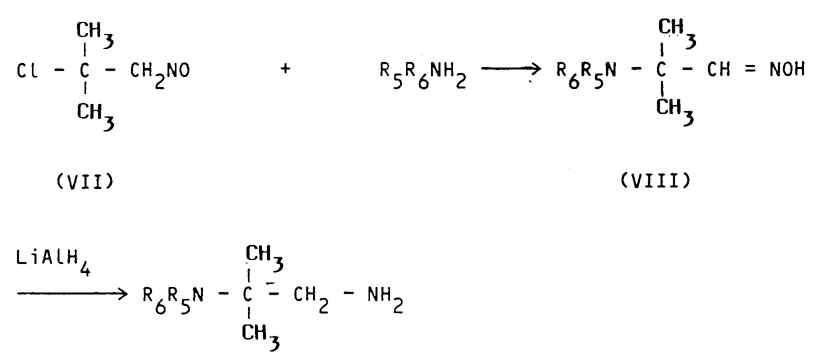

Le composé de formule (VII) peut être utilisé sous forme d'un dimère (VII bis), qui est obtenu par action du chlorure de nitrosyle sur l'oléfine appropriée (IX) selon le mode opératoire décrit dans J. Prakt. Chem., 1965, 29 (4), 123

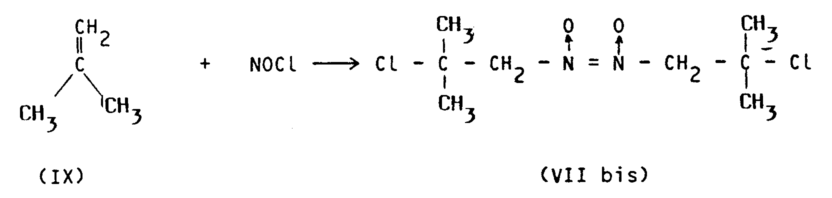

Les exemples suivants illustrent l'invention. Les composés sont caractérisés par leur point de fusion (F) exprimé en degrés Celcius.

### Exemple 1

Sesquifumarate de (diéthylamino-2 méthyl-2 propyl)amino-3 phényl-6 propyl-5 pyridazine : SR 46559 A.

### A) Chloro-6 phényl-3 propyl-4 pyridazine.

1. Hydroxy-2 oxo-4 phényl-4 propyl-3 butyrate d'éthyle.
   On chauffe à 120°C, pendant 15 heures, le mélange de 48,67 g de valérophénone et 45,94 g de glyoxylate d'éthyle.
   Le produit brut de réaction est utilisé tel quel pour l'opération suivante.
2. Phényl-6 propyl-5 2H-pyridazinone-3.
   On dissout le produit brut obtenu ci-dessus dans 450 ml de n-butanol, puis on ajoute 30 g d'hydrate d'hydrazine et chauffe au reflux pendant 24 heures.
   On concentre le n-butanol sous vide. On reprend le résidu dans un mélange de 300 ml d'acide acétique et 30 ml d'acide chlorhydrique concentré. On chauffe le mélange à 100°C pendant 3 heures. On verse la solution dans l'eau froide et laisse cristalliser.
   On essore le solide et sèche.
   Poids : 44 g.
   F : 160°C.
   Rendement : 69 %.
3. Chloro-6 phényl-3 propyl-4 pyridazine.
   A 44 g de pyridazinone obtenue ci-dessus on ajoute 250 ml d'oxychlorure de phosphore et chauffe à 80°C pendant 4 heures.
   Après une nuit à température ambiante, on concentre le milieu réactionnel aux 3/4 puis on verse lentement le mélange sur de la glace. On extrait 2 fois par 300 ml de dichlorométhane, sèche sur sulfate de sodium et concentre. On effectue ensuite une chromatographie sur silice en éluant par un mélange acétate d'éthylechlorure de méthylène (50/50, v/v). Après recristallisation dans l'éther isopropylique, on obtient 43,7 g du produit attendu.
   F : 60°C.
   Rendement : 92 %.

### B) Préparation de la diéthylamino-2 méthyl-2 propylamine.

1. Diéthylamino-2 méthyl-2 propionitrile.
   On mélange 85,1 g de cyanhydrine de l'acétone distillée et 73,1 g de diéthylamine puis on ajoute 85,7 g de sulfate de magnésium et l'on chauffe sous léger reflux pendant 20 heures sous agitation. On filtre le bloc de sulfate qui s'est formé puis on le lave à l'éther. Le filtrat est concentré puis distillé.
   On recueille 86,6 g du produit attendu.
   Rendement : 62 %.
   Eb : 68-70°C sous 1998 Pa (15 mm de mercure).
2. Diéthylamino-2 méthyl-2 propionamide.
   A 95,9 g du nitrile préparé à l'étape précédente, on ajoute sous agitation 450 ml d'acide sulfurique et 70 ml d'eau et on chauffe dans un bain d'huile à 100-110°C pendant 2 heures. On verse le mélange réactionnel lentement, en 1 heure, dans 1,4 l d'une solution d'ammoniaque à 20 % et 400 ml d'eau en refroidissant par un bain de carboglace dans l'acétone. On extrait 3 fois par 600 ml de chlorure de méthylène, sèche sur sulfate de sodium et concentre.
   Le produit attendu est obtenu par distillation.
   Poids : 102,5 g.
   Rendement : 95 %.
   Eb : 134-139°C sous 1998 Pa (15 mm de mercure).
3. Diéthylamino-2 méthyl-2 propylamine.
   On chauffe à 45-50°C un mélange contenant 52,4 g de l'amide préparé à l'étape précédente et 60 ml de tétrahydrofuranne. On ajoute sous atmosphère d'azote, en 1 heure, 86 ml du complexe borane-diméthylsulfure et on continue à chauffer pendant 3 heures dans un bain d'huile à 80-85°C.
   Après une nuit à température ambiante, on refroidit dans un bain glacé puis on ajoute très lentement, en 3 heures, 315 ml d'acide chlorhydrique 6N et on chauffe à nouveau à 135°C pendant 3 heures. Après une nuit à température ambiante, on ajoute 200 ml de soude à 30 %, tout en refroidissant le mélange réactionnel. On extrait 3 fois par 250 ml d'éther puis on sèche sur sulfate de sodium et concentre.
   Le produit attendu est obtenu par distillation.
   Poids : 23 g.
   Rendement : 48 %.
   Eb = 71-73°C sous 1998 Pa (15 mm de mercure).
C) SR 46559 A
   On chauffe à 120°C pendant une nuit le mélange de 2,5 g du dérivé chloré obtenu ci-dessus à l'étape A et 4,6 g de la diamine obtenue à l'étape B. On ajoute 150 ml d'acétate d'éthyle puis extrait 2 fois par 50 ml d'acide chlorhydrique. On alcalinise ensuite par addition de 50 ml de soude à 30 % puis extrait par de l'acétate d'éthyle. On lave à l'eau salée, sèche sur sulfate de sodium et concentre. On effectue une chromatographie sur alumine en éluant par un mélange chlorure de méthylène-acétate d'éthyle (70/30, v/v).
   On obtient 3,2 g d'une huile qui cristallise.
   F : 75-77°C.
   Rendement : 87 %.

### Sesquifumarate

On reprend 3,1 g de la base obtenue précédemment dans 50 ml d'acétone et on ajoute 1,6 g d'acide fumarique dans 150 ml d'acétone. On filtre à chaud. Le volume total recueilli (175 ml) est concentré jusqu'à 130 ml. On laisse cristalliser, on filtre les cristaux puis on les lave à l'acétone.
On obtient 4,1 g du produit attendu.
Rendement global de l'étape E : 74 %.
F = 151°C.

### Exemple 2

### SR 46559 A

A) Chloro-6 phényl-3 propyl-4 pyridazine, décrit à l'exemple 1.
B) Diéthylamino-2 méthyl-2 propylamine.

1. Préparation du composé de formule (VII bis).
   47,14 g d'isobutylène sont dissous dans 150 ml de n-heptane, on refroidit le mélange à une température comprise entre -10° et -20°C et l'on ajoute 50 g de chlorure de nitrosyle. On laisse remonter la température (+5°C) pendant 1 heure et demie puis la température est portée entre 10°C et 20°C et le mélange est laissé sous agitation pendant 1 heure et demie. Le précipité formé est filtré, lavé à l'heptane puis séché.
   F = 102-104°C.
   m : 64 g.
2. Préparation du composé de formule VIII : R₅ = R₆ = C₂H₅
   21,7 g du composé préparé à l'étape précédente sont mis en suspension dans 150 ml d'alcool absolu, on ajoute 39,17 g de diéthylamine et on chauffe à 60°C pendant 6 heures. On obtient une huile qui se solidifie.
   m = 19,5 g.
   F > 50°C.
3. Diéthylamino-2 méthyl-2 propylamine.
   A 50 ml d'une solution éthérée du composé obtenu à l'étape précédente, on ajoute 7,01 g d'hydrure de lithium et d'aluminium en 1 heure. Après 1 heure et demie d'agitation à température ambiante, on porte à reflux pendant 4 heures. Le mélange étant maintenu entre 0°C et -10°C, on ajoute ensuite 7,1 ml d'eau en 1 heure, 7,1 ml de soude en 30 minutes et 21,3 ml d'eau en 30 minutes. Après 2 heures sous agitation à température ambiante, on filtre la solution, lave à l'éther anhydre, sèche le filtrat sur sulfate de sodium puis concentre les solvants. Le produit est distillé.
   Eb = 72-75°C sous 1998 Pa (15 mm de mercure).
   m : 4,2 g.
C) On prépare ensuite le SR 46559 A comme décrit à l'exemple 1.

### Exemple 3

### Sesquifumarate de (diéthylamino-2 méthyl-2 propyl)amino-3 (chloro-2 phényl)-6 propyl-5 pyridazine. SR 47863 A.

1,7 g de chloro-3 (chloro-2 phényl)-6 propyl-5 pyridazine et 6 ml de diéthylamino-2 méthyl-2 propylamine sont chauffés à 110°C, sous azote, pendant 20 heures.

Après évaporation sous vide, le mélange est repris dans le dichlorométhane et lavé avec une solution de carbonate de sodium. La phase organique est décantée, séchée sur sulfate de magnésium, filtrée et concentrée sous vide. Le résidu est chromatographié sur gel de silice, éluant : dichlorométhane/méthanol 98/2.

La concentration des fractions pures fournit une huile qui est dissoute dans 10 ml de méthanol. On ajoute de l'acide fumarique, le méthanol est concentré sous vide et le sesquifumarate cristallise dans l'éther.
m = 1,6 g.
F = 144°C.

### Exemple 4

### (diéthylamino-2 méthyl-2 propyl)amino-3 (méthoxy-2 phényl)-6 méthyl-5 pyridazine.

1,6 g de chloro-3 (méthoxy-2 phényl)-6 méthyl-5 pyridazine, 4 g de diéthylamino-2 méthyl-2 propylamine et 0,36 g de chlorure d'ammonium sont cofondus à 120°C et le mélange réactionnel est laissé à cette température pendant 24 heures.

Le mélange est refroidi à température ambiante, extrait à l'acétate d'éthyle et lavé avec une solution aqueuse saturée de chlorure de sodium.

La phase organique est décantée, séchée sur MgSO₄, filtrée et concentrée sous vide.

Le résidu est chromatographié sur alumine, éluant : acétate d'éthyle +2 % de triéthylamine.

La concentration des fractions pures fournit le produit attendu. La structure est vérifiée par l'analyse du spectre de RMN.

### Exemple 5

### (diéthylamino-2 méthyl-2 propyl)amino-3 méthyl-5 (hydroxy-2 phényl)-6 pyridazine. SR 96376.

1 g du produit obtenu précédemment à l'exemple 4 est mis en solution dans 50 ml d'acide bromhydrique à 48 % et le mélange est chauffé à reflux pendant 48 heures. Après ce temps, le mélange réactionnel est concentré sous vide, le résidu est alcalinisé avec une solution aqueuse de carbonate de potassium et la solution est extraite au dichlorométhane. La phase organique est décantée, séchée sur MgSO₄, filtrée et concentrée sous vide.

Le résidu est chromatographié sur alumine, éluant : acétate d'éthyle/méthanol 9/1 + 2 % de triéthylamine.

La concentration des fractions pures fournit un résidu qui est recristallisé dans de l'isopropanol.
m = 200 mg.
F = 159,2°C.

### Exemples 6 à 16

A) En opérant comme indiqué dans l'exemple 1A, mais en faisant varier la cétone de départ, on obtient les chloro-6 pyridazines réunies dans le tableau 1. 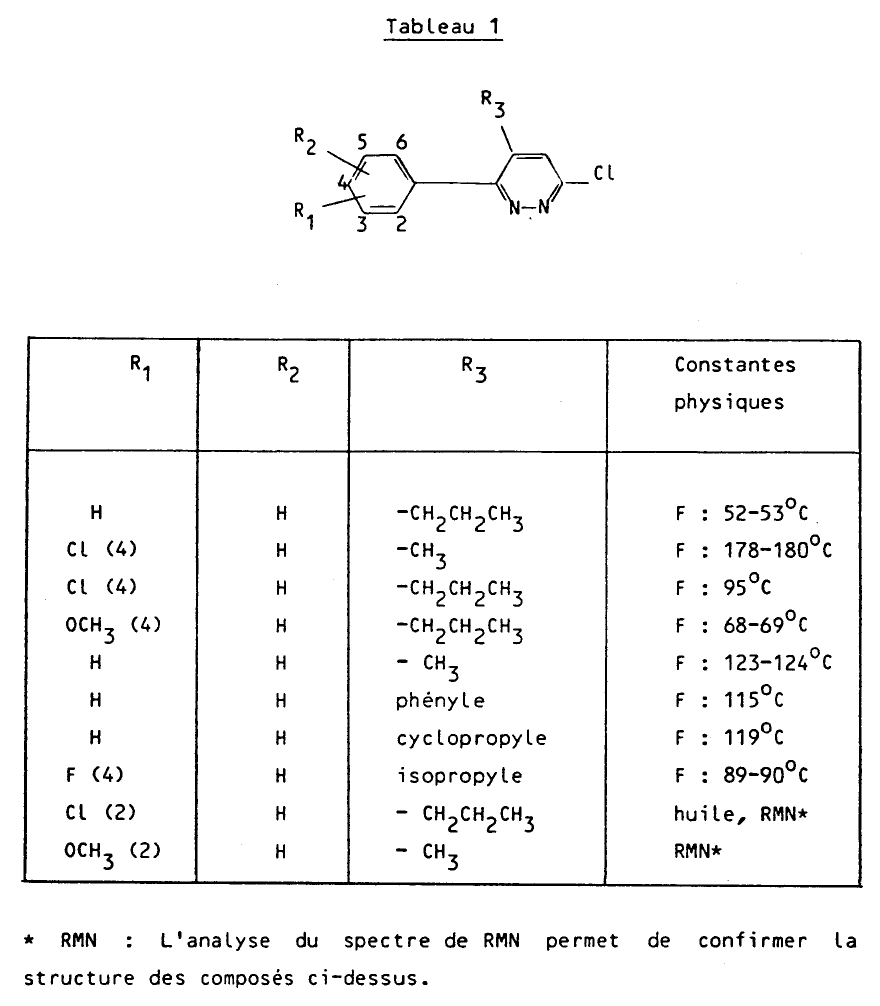
B) A partir des dérivés chlorés du tableau 1, en faisant varier les amines NH₂R₄ utilisées, on obtient, en suivant le mode opératoire de l'exemple 1, les composés selon l'invention rassemblés dans le tableau 2 ci-dessous.

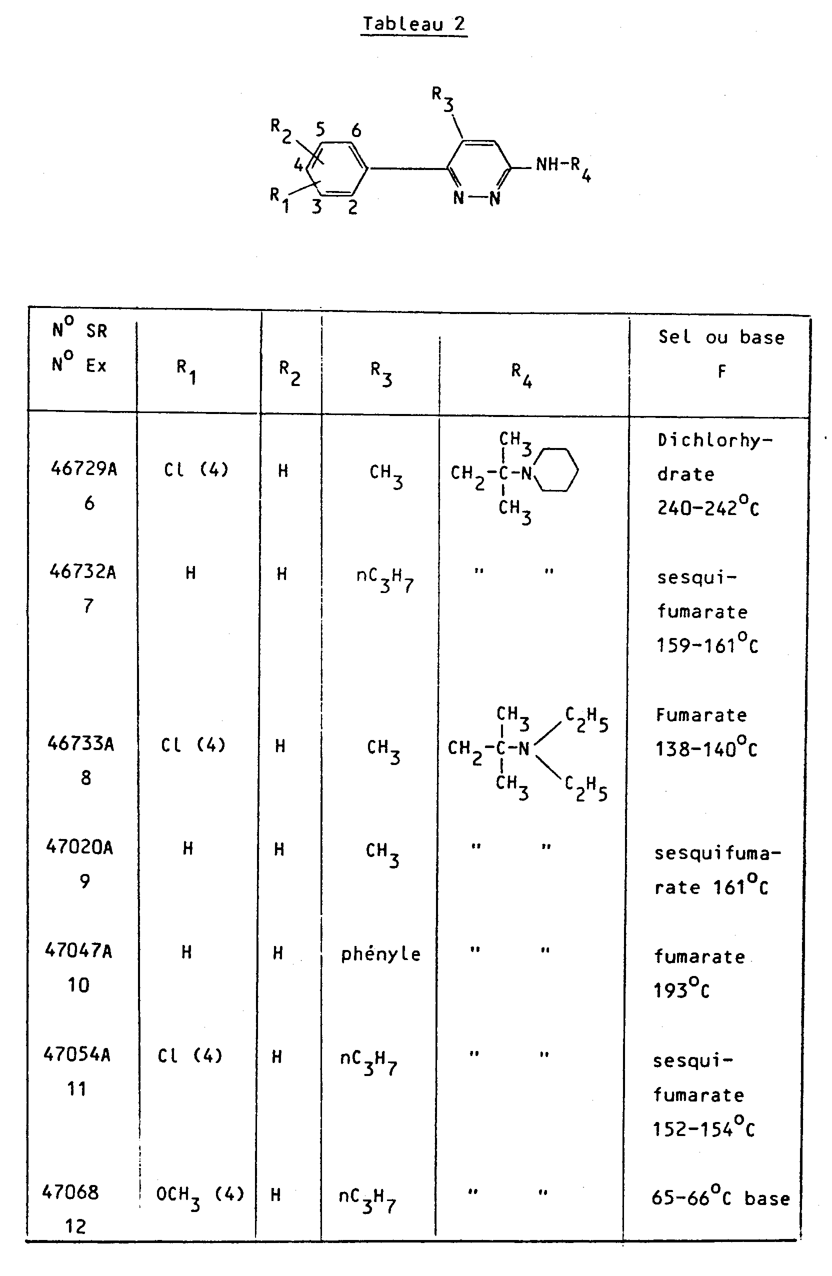

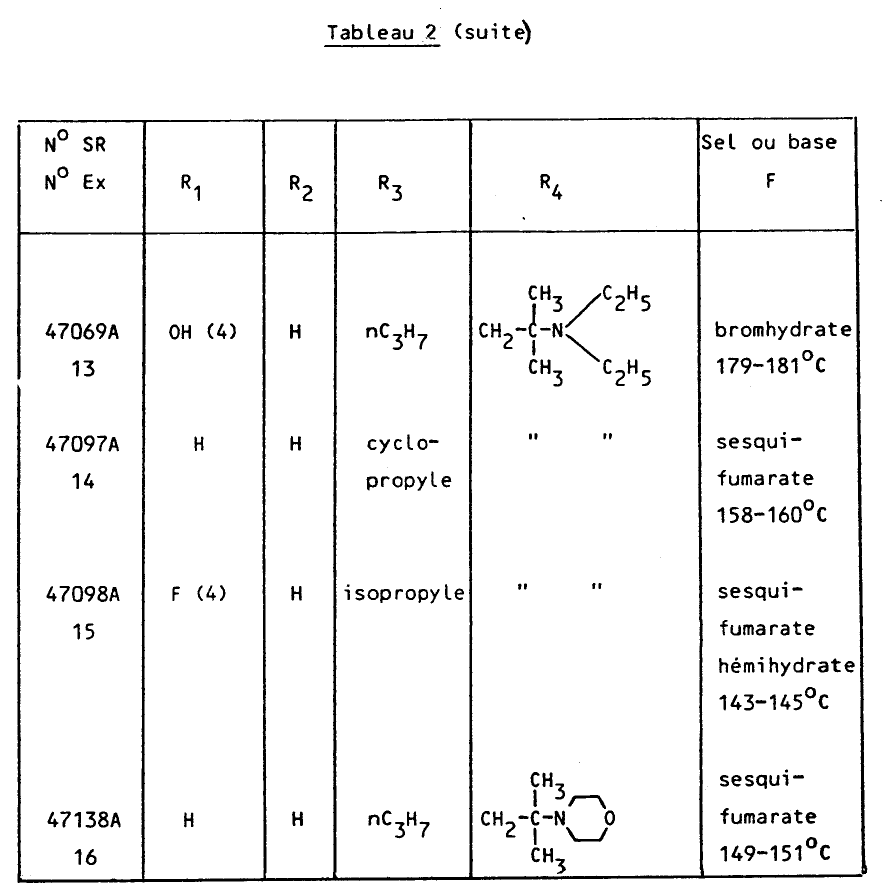

Les produits selon l'invention ont été étudiés en ce qui concerne leurs propriétés pharmacologiques et en particulier en ce qui concerne leur affinité pour les récepteurs cholinergiques muscariniques de type M₁ et M₂.

In vitro, les composés selon l'invention ont été essayés suivant la technique décrite par Watson J.D. et al. (Life Science, 1982, 31, 2019-2029) en ce qui concerne leur affinité pour les récepteurs de type M₁ et selon la technique décrite par Hammer R. et al. (Nature, 1980, 283, 90-92) et Hulme E.C. et al. (Molecular Pharmacology, 1978, 14, 737-750), en ce qui concerne leur affinité pour les récepteurs de type M₂.

Les composés selon l'invention présentent une bonne affinité pour les récepteurs de type M₁ et une spécificité marquée pour les récepteurs centraux de type M₁ vis-à-vis des récepteurs de type M₂.

A titre d'exemple, le composé SR 46559 A a montré une concentration inhibitrice 50 exprimée en micromoles de 0,11 et 2,2 respectivement sur les récepteurs M₁ et M₂.

De même, le composé SR 47047 A a montré des concentrations inhibitrices 50 de 0,04 et 0,9 respectivement sur les récepteurs M₁ et M₂.

In vivo, les composés selon l'invention ont été essayés sur le test des rotations induites par la pirenzépine intrastriatale décrit par Worms P. et al. (Psychopharmacology, 1987, 93, 489-493) modifié en ce que l'administration des produits par voie orale a eu lieu 4 heures avant, au lieu de 30 minutes avant, l'injection de pirenzépine.

A la dose de 3 mg par kg de poids corporel, les produits selon l'invention inhibent fortement le nombre des rotations induites par la pirenzépine. Ainsi à titre d'exemple, le composé SR 46559 A inhibe de 78 % les rotations induites par la pirenzépine.

De plus, les composés selon l'invention se sont montrés actifs sur les tests d'évitement passif chez le rat, décrit par Jarvik M.E. et al. dans Psychol. Med., 1967, 21, 221-224 et par Worms P. et al. dans Psychopharmacol., 1989, 98, 286-288.

Ainsi, d'après les résultats de ces tests, les produits selon l'invention s'opposent à l'amnésie induite par la scopolamine administrée par voie intrapéritonéale à 0,5 mg/kg et à l'amnésie induite par la pirenzépine administrée par voie intrapéritonéale à 75 mg/kg. Par exemple, le SR 46559 A présente respectivement une dose efficace 50 de 0,25 mg/kg per os et de 0,027 mg/kg per os dans chacun de ces tests.

Par ailleurs, certains composés selon l'invention ont été étudiés dans plusieurs modèles prédictifs d'une activité anti-dépressive comme le test de la nage forcée décrit par Porsolt et al. (Arch. Intern. Pharmacodyn., 1977, 229, 327-336) et le test d'antagonisme de la ptôse à la réserpine décrit par Gouret et al. (J. Pharmacol. (Paris), 1977, 8, 333-350). Le SR 46559 A notamment s'est montré inactif dans ces tests à des doses variant de 0,1 à 10 mg/kg per os.

Enfin, les composés selon l'invention n'ont mis en évidence aucun signe de toxicité aux doses où ils sont actifs.

Par suite, les composés selon l'invention peuvent être utilisés en tant que médicaments.

Les résultats indiqués montrent que les composés selon l'invention présentent une bonne affinité pour les récepteurs muscariniques et une bonne activité dans les tests d'amnésie induite par la scopolamine ou la pirenzépine. Ils permettent d'envisager l'utilisation des produits selon l'invention dans tous les cas où se manifeste un déficit cholinergique et notamment pour le traitement des troubles mnésiques cognitifs, des syndromes dégénératifs liés à la sénescence et aux démences séniles.

Selon un autre de ses aspects, la présente demande concerne donc les compositions pharmaceutiques contenant au moins un des composés selon l'invention ou un de leurs sels en tant qu'ingrédient actif.

Dans les compositions pharmaceutiques de la présente invention pour l'administration orale, sublinguale, transdermique ou rectale, les ingrédients actifs ci-dessus peuvent être administrés sous forme unitaire d'administration, en mélange avec les supports pharmaceutiques classiques, aux êtres humains notamment pour le traitement des troubles mnésiques cognitifs ou des syndromes dégénératifs. Les formes unitaires d'administration appropriées comprennent les formes par voie orale, telles que les comprimés, les gélules, les poudres, les granulés et les solutions ou suspensions orales, les forme d'administration sublinguale et buccale, les formes d'administration sous-cutanée, intramusculaire ou intraveineuse et les formes d'administration rectale.

Afin d'obtenir l'effet désiré, la dose de principe actif peut varier entre 0,5 et 500 mg par jour.

Chaque dose unitaire peut contenir de 0,1 à 100 mg d'ingrédient actif en combinaison avec un support pharmaceutique. Cette dose unitaire peut être administrée 1 à 5 fois par jour.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans les gélules molles ou dures.

Les poudres ou les granulés dispersibles dans l'eau peuvent contenir l'ingrédient actif en mélange avec des agents de dispersion ou des agents mouillants, ou des agents de mise en suspension comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol et le butylèneglycol.

Le principe actif peut être formulé également sous forme de microcapsules, éventuellement avec un ou plusieurs supports ou additifs.

A titre de préparation galénique, on peut préparer des gélules contenant :
- SR 46559 A: 0,010 g
- Lactose: 0,050 g
- Stéarate de magnésium: 0,005 g
en mélangeant intimement les ingrédients ci-dessus et en versant le mélange dans des gélules de gélatine dure.

## Revendications (Revendications pour l'(les) Etat(s) contractant(s) suivant(s): AT, BE, CH, LI, DE, DK, FR, GB, IT, LU, NL, SE)

1. Dérivé de la pyridazine, choisi parmi :
* la 3-(2-diéthylamino-2-méthylpropyl)amino-6-phényl-5-propylpyridazine ;
* la 3-(2-diéthylamino-2-méthylpropyl)amino-6-(2-chlorophényl)-5-propylpyridazine ;
* la 3-(2-diéthylamino-2-méthylpropyl)amino-6-(2-méthoxyphényl)-5-méthylpyridazine ;
* la 3-(2-diéthylamino-2-méthylpropyl)amino-6-(2-hydroxyphényl)-5-méthylpyridazine ;
* la 3-(2-pipéridino-2-méthylpropyl)amino-6-(4-chlorophényl)-5-méthylpyridazine ;
* la 3-(2-pipéridino-2-méthylpropyl)amino-6-phényl-5-propylpyridazine ;
* la 3-(2-diéthylamino-2-méthylpropyl)amino-6-(4-chlorophényl)-5-méthylpyridazine ;
* la 3-(2-diéthylamino-2-méthylpropyl)amino-6-phényl-5-méthylpyridazine ;
* la 3-(2-diéthylamino-2-méthylpropyl)amino-5,6-diphénylpyridazine ;
* la 3-(2-diéthylamino-2-méthylpropyl)amino-6-(4-chlorophényl)-5-propylpyridazine ;
* la 3-(2-diéthylamino-2-méthylpropyl)amino-6-(4-méthoxyphényl)-5-propylpyridazine ;
* la 3-(2-diéthylamino-2-méthylpropyl)amino-6-(4-hydroxyphényl)-5-propylpyridazine ;
* la 3-(2-diéthylamino-2-méthylpropyl)amino-5-cyclopropyl-6-phénylpyridazine ;
* la 3-(2-diéthylamino-2-méthylpropyl)amino-5-isopropyl-6-(4-fluorophényl)pyridazine ;
* la 3-(2-(morpholin-4-yl)-2-méthylpropyl)amino-6-phényl-5-propylpyridazine ;
et leurs sels avec des acides minéraux ou organiques.

2. Dérivé selon la revendication 1, qui est la 3-(2-diéthylamino-2-méthylpropyl)amino-6-phényl-5-propylpyridazine ou l'un de ses sels avec des acides minéraux ou organiques.

3. Dérivé selon la revendication 1, qui est la 3-(2-diéthylamino-2-méthylpropyl)amino-5,6-diphénylpyridazine ou l'un de ses sels avec des acides minéraux ou organiques.

4. Procédé de préparation des dérivés selon la revendication 1, caractérisé en ce que l'on fait réagir une amine de formule R₄NH₂ dans laquelle R₄ représente un groupe 2-diéthylamino-2-méthylpropyle ou le cas échéant 2-pipéridino-2-méthylpropyle ou 2-(morpholin-4-yl)-2-méthylpropyle, avec une chloro-6-pyridazine de formule : 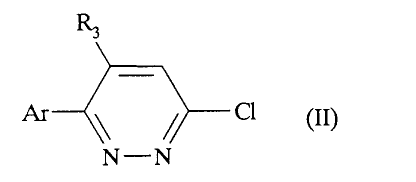 dans laquelle :
* R₃ représente un propyle et Ar représente un phényle ou le cas échéant un 2-chlorophényle, un 4-chlorophényle, un 4-méthoxyphényle ou un 4-hydroxyphényle ;
ou
* R₃ représente un méthyle et Ar représente un 2-méthoxyphényle ou le cas échéant un 2-hydroxyphényle, un 4-chlorophényle ou un phényle ;
ou
* R₃ représente un phényle et Ar représente un phényle ;
ou
* R₃ représente un cyclopropyle et Ar un phényle ;
ou
* R₃ représente un isopropyle et Ar un 4-fluorophényle ; et éventullement on salifie le composé ainsi obtenu avec un acide minéral ou organique.

5. Composition pharmaceutique, caractérisée en ce qu'elle contient comme principe actif un dérivé selon l'une quelconque des revendications 1 à 3.

6. Composition pharmaceutique selon la revendication 5, caractérisée en ce qu'elle contient comme principe actif le dérivé selon la revendication 2.

7. Composition pharmaceutique selon la revendication 5, caractérisée en ce qu'elle contient comme principe actif le dérivé selon la revendication 3.

8. Composition pharmaceutique selon l'une quelconque des revendications 5 à 7, caractérisée en ce qu'elle comprend de 0,1 à 100 mg de principe actif par unité de dosage.

## Revendications (Revendications pour l'(les) Etat(s) contractant(s) suivant(s): ES)

1. Procédé pour la préparation d'un dérivé de pyridazine choisi parmi :
* la 3-(2-diéthylamino-2-méthylpropyl)amino-6-phényl-5-propylpyridazine ;
* la 3-(2-diéthylamino-2-méthylpropyl)amino-6-(2-chlorophényl)-5-propylpyridazine ;
* la 3-(2-diéthylamino-2-méthylpropyl)amino-6-(2-méthoxyphényl)-5-méthylpyridazine ;
* la 3-(2-diéthylamino-2-méthylpropyl)amino-6-(2-hydroxyphényl)-5-méthylpyridazine ;
* la 3-(2-pipéridino-2-méthylpropyl)amino-6-(4-chlorophényl)-5-méthylpyridazine ;
* la 3-(2-pipéridino-2-méthylpropyl)amino-6-phényl-5-propylpyridazine ;
* la 3-(2-diéthylamino-2-méthylpropyl)amino-6-(4-chlorophényl)-5-méthylpyridazine ;
* la 3-(2-diéthylamino-2-méthylpropyl)amino-6-phényl-5-méthylpyridazine ;
* la 3-(2-diéthylamino-2-méthylpropyl)amino-5,6-diphénylpyridazine ;
* la 3-(2-diéthylamino-2-méthylpropyl)amino-6-(4-chlorophényl)-5-propylpyridazine ;
* la 3-(2-diéthylamino-2-méthylpropyl)amino-6-(4-méthoxyphényl)-5-propylpyridazine ;
* la 3-(2-diéthylamino-2-méthylpropyl)amino-6-(4-hydroxyphényl)-5-propylpyridazine ;
* la 3-(2-diéthylamino-2-méthylpropyl)amino-5-cyclopropyl-6-phénylpyridazine ;
* la 3-(2-diéthylamino-2-méthylpropyl)amino-5-isopropyl-6-(4-fluorophényl)pyridazine ;
* la 3-(2-(morpholin-4-yl)-2-méthylpropyl)amino-6-phényl-5-propylpyridazine ;
ou d'un de leurs sels avec des acides minéraux ou organiques, caractérisé en ce que l'on fait réagir une amine de formule R₄NH₂ dans laquelle R₄ représente un groupe 2-diéthylamino-2-méthylpropyle ou le cas échéant 2-pipéridino-2-méthylpropyle ou 2-(morpholin-4-yl)-2-méthylpropyle, avec une chloro-6-pyridazine de formule : 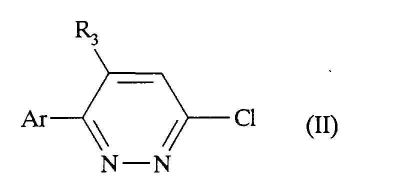 dans laquelle :
* R₃ représente un propyle et Ar représente un phényle ou le cas échéant un 2-chlorophényle, un 4-chlorophényle, un 4-méthoxyphényle ou un 4-hydroxyphényle ;
ou
* R₃ représente un méthyle et Ar représente un 2-méthoxyphényle ou le cas échéant un 2-hydroxyphényle, un 4-chlorophényle ou un phényle ;
ou
* R₃ représente un phényle et Ar représente un phényle ;
ou
* R₃ représente un cyclopropyle et Ar un phényle ;
ou
* R₃ représente un isopropyle et Ar un 4-fluorophényle ; et éventullement on salifie le composé ainsi obtenu avec un acide minéral ou organique.

2. Procédé selon la revendication 1, pour la préparation de la 3-(2-diéthylamino-2-méthylpropyl)amino-6-phényl-5-propylpyridazine, caractérisé en ce qu'on fait réagir une amine R₄NH₂ dans laquelle R₄ représente un groupe 2-diéthylamino-2-méthylpropyle avec une chloro-6-pyridazine (II) dans laquelle R₃ représente un propyle et Ar un phényle.

3. Procédé selon la revendication 1, pour la préparation de la 3-(2-diéthylamino-2-méthylpropyl)amino-5,6-diphénylpyridazine, caractérisé en ce qu'on fait réagir une amine R₄NH₂ dans laquelle R₄ représente un groupe 2-diéthylamino-2-méthylpropyle avec une chloro-6-pyridazine (II) dans laquelle R₃ et Ar représentent chacun un phényle.

4. Procédé pour la préparation d'une composition pharmaceutique contenant comme principe actif un dérivé obtenu par le procédé de l'une quelconque des revendications 1 à 3, caractérisé en ce qu'il consiste à mélanger ledit dérivé avec au moins un excipient pharmaceutiquement acceptable.

5. Procédé selon la revendication 4, caractérisé en ce qu'on utilise comme principe actif le dérivé obtenu selon le procédé de la revendication 2.

6. Procédé selon la revendication 4, caractérisé en ce qu'on utilise comme principe actif le dérivé obtenu selon le procédé de la revendication 3.

7. Procédé selon l'une quelconque des revendications 4 à 6, caractérisé en ce que la composition pharmaceutique contient de 0,1 à 100 mg de principe actif par unité de dosage.

## Revendications (Revendications pour l'(les) Etat(s) contractant(s) suivant(s): GR)

1. Dérivé de la pyridazine, choisi parmi :
* la 3-(2-diéthylamino-2-méthylpropyl)amino-6-phényl-5-propylpyridazine ;
* la 3-(2-diéthylamino-2-méthylpropyl)amino-6-(2-chlorophényl)-5-propylpyridazine ;
* la 3-(2-diéthylamino-2-méthylpropyl)amino-6-(2-méthoxyphényl)-5-méthylpyridazine ;
* la 3-(2-diéthylamino-2-méthylpropyl)amino-6-(2-hydroxyphényl)-5-méthylpyridazine ;
* la 3-(2-pipéridino-2-méthylpropyl)amino-6-(4-chlorophényl)-5-méthylpyridazine ;
* la 3-(2-pipéridino-2-méthylpropyl)amino-6-phényl-5-propylpyridazine ;
* la 3-(2-diéthylamino-2-méthylpropyl)amino-6-(4-chlorophényl)-5-méthylpyridazine ;
* la 3-(2-diéthylamino-2-méthylpropyl)amino-6-phényl-5-méthylpyridazine ;
* la 3-(2-diéthylamino-2-méthylpropyl)amino-5,6-diphénylpyridazine ;
* la 3-(2-diéthylamino-2-méthylpropyl)amino-6-(4-chlorophényl)-5-propylpyridazine ;
* la 3-(2-diéthylamino-2-méthylpropyl)amino-6-(4-méthoxyphényl)-5-propylpyridazine ;
* la 3-(2-diéthylamino-2-méthylpropyl)amino-6-(4-hydroxyphényl)-5-propylpyridazine ;
* la 3-(2-diéthylamino-2-méthylpropyl)amino-5-cyclopropyl-6-phénylpyridazine ;
* la 3-(2-diéthylamino-2-méthylpropyl)amino-5-isopropyl-6-(4-fluorophényl)pyridazine ;
* la 3-(2-(morpholin-4-yl)-2-méthylpropyl)amino-6-phényl-5-propylpyridazine ;
et leurs sels avec des acides minéraux ou organiques.

2. Dérivé selon la revendication 1, qui est la 3-(2-diéthylamino-2-méthylpropyl)amino-6-phényl-5-propylpyridazine ou l'un de ses sels avec des acides minéraux ou organiques.

3. Dérivé selon la revendication 1, qui est la 3-(2-diéthylamino-2-méthylpropyl)amino-5,6-diphénylpyridazine ou l'un de ses sels avec des acides minéraux ou organiques.

4. Procédé de préparation des dérivés selon la revendication 1, caractérisé en ce que l'on fait réagir une amine de formule R₄NH₂ dans laquelle R₄ représente un groupe 2-diéthylamino-2-méthylpropyle ou le cas échéant 2-pipéridino-2-méthylpropyle ou 2-(morpholin-4-yl)-2-méthylpropyle, avec une chloro-6-pyridazine de formule : 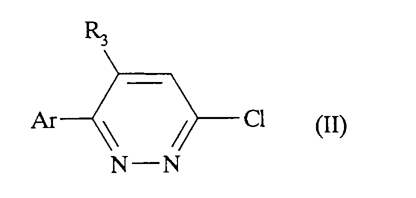 dans laquelle :
* R₃ représente un propyle et Ar représente un phényle ou le cas échéant un 2-chlorophényle, un 4-chlorophényle, un 4-méthoxyphényle ou un 4-hydroxyphényle ;
ou
* R₃ représente un méthyle et Ar représente un 2-méthoxyphényle ou le cas échéant un 2-hydroxyphényle, un 4-chlorophényle ou un phényle ;
ou
* R₃ représente un phényle et Ar représente un phényle ;
ou
* R₃ représente un cyclopropyle et Ar un phényle ;
ou
* R₃ représente un isopropyle et Ar un 4-fluorophényle ; et éventullement on salifie le composé ainsi obtenu avec un acide minéral ou organique.

5. Procédé pour la préparation d'une composition pharmaceutique contenant comme principe actif un dérivé selon l'une quelconque des revendications 1 à 3, caractérisé en ce qu'il consiste à mélanger ledit dérivé avec au moins un excipient pharmaceutiquement acceptable.

6. Procédé selon la revendication 5, caractérisé en ce qu'on utilise comme principe actif le dérivé selon la revendication 2.

7. Procédé selon la revendication 5, caractérisé en ce qu'on utilise comme principe actif le dérivé selon la revendication 3.

8. Procédé selon l'une quelconque des revendications 5 à 7, caractérisé en ce que la composition pharmaceutique contient de 0,1 g à 100 mg de principe actif par unité de dosage.

## Claims (Claims for the following Contracting State(s): AT, BE, CH, LI, DE, DK, FR, GB, IT, LU, NL, SE)

1. Pyridazine derivative, selected from:
* 3-(2-diethylamino-2-methylpropyl)amino-6-phenyl-5-propylpyridazine;
* 3-(2-diethylamino-2-methylpropyl)amino-6-(2-chlorophenyl)-5-propylpyridazine;
* 3-(2-diethylamino-2-methylpropyl)amino-6-(2-methoxyphenyl)-5-methylpyridazine;
* 3-(2-diethylamino-2-methylpropyl)amino-6-(2-hydroxyphenyl)-5-methylpyridazine;
* 3-(2-piperidino-2-methylpropyl)amino-6-(4-chlorophenyl)-5-methylpyridazine;
* 3-(2-piperidino-2-methylpropyl)amino-6-phenyl-5-propylpyridazine;
* 3-(2-diethylamino-2-methylpropyl)amino-6-(4-chlorophenyl)-5-methylpyridazine;
* 3-(2-diethylamino-2-methylpropyl)amino-6-phenyl-5-methylpyridazine;
* 3-(2-diethylamino-2-methylpropyl)amino-5,6-diphenylpyridazine;
* 3-(2-diethylamino-2-methylpropyl)amino-6-(4-chlorophenyl)-5-propylpyridazine;
* 3-(2-diethylamino-2-methylpropyl)amino-6-(4-methoxyphenyl)-5-propylpyridazine;
* 3-(2-diethylamino-2-methylpropyl)amino-6-(4-hydroxyphenyl)-5-propylpyridazine;
* 3-(2-diethylamino-2-methylpropyl)amino-5-cyclopropyl-6-phenylpyridazine;
* 3-(2-diethylamino-2-methylpropyl)amino-5-isopropyl-6-(4-fluorophenyl)pyridazine;
* 3-(2-(morpholin-4-yl)-2-methylpropyl)amino-6-phenyl-5-propylpyridazine;
and the salts thereof with mineral or organic acids.

2. Derivative according to claim 1, which is the 3-(2-diethylamino-2-methylpropyl)amino-6-phenyl-5-propylpyridazine or a salt thereof with mineral or organic acids.

3. Derivative according to claim 1, which is the 3-(2-diethylamino-2-methylpropyl)amino-5,6-diphenylpyridazine or a salt thereof with mineral or organic acids.

4. Process for the preparation of the derivatives according to claim 1, characterized in that an amine of formula R₄NH₂ in which R₄ is a 2-diethylamino-2-methylpropyl or, if need be, 2-piperidino-2-methylpropyl or 2-(morpholin-4-yl)-2-methylpropyl group, is reacted with a 6-chloropyridazine of formula: 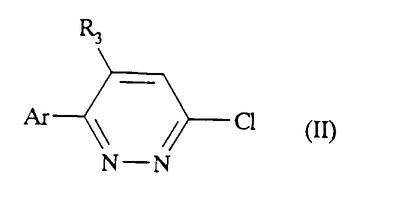 in which:
* R₃ is a propyl and Ar is a phenyl or, if need be, a 2-chlorophenyl, a 4-chlorophenyl, a 4-methoxyphenyl or a 4-hydroxyphenyl;
or
* R₃ is a methyl and Ar is a 2-methoxyphenyl or, if need be, a 2-hydroxyphenyl, a 4-chlorophenyl or a phenyl;
or
* R₃ is a phenyl and Ar is a phenyl;
or
* R₃ is a cyclopropyl and Ar a phenyl;
or
* R₃ is an isopropyl and Ar a 4-fluorophenyl;
and, optionally, the compound thus obtained is converted into a salt with a mineral or organic acid.

5. Pharmaceutical composition, characterized in that it contains as active principle a derivative according to any one of claims 1 to 3.

6. Pharmaceutical composition according to claim 5, characterized in that it contains as active principle the derivative according to claim 2.

7. Pharmaceutical composition according to claim 5, characterized in that it contains as active principle the derivative according to claim 3.

8. Pharmaceutical composition according to any one of claims 5 to 7, characterized in that it contains from 0.1 to 100 mg of active principle per dosage unit.

## Claims (Claims for the following Contracting State(s): ES)

1. Process for the preparation of a pyridazine derivative selected from:
* 3-(2-diethylamino-2-methylpropyl)amino-6-phenyl-5-propylpyridazine;
* 3-(2-diethylamino-2-methylpropyl)amino-6-(2-chlorophenyl)-5-propylpyridazine;
* 3-(2-diethylamino-2-methylpropyl)amino-6-(2-methoxyphenyl)-5-methylpyridazine;
* 3-(2-diethylamino-2-methylproplyl)amino-6-(2-hydroxyphenyl)-5-methylpyridazine;
* 3-(2-piperidino-2-methylpropyl)amino-6-(4-chlorophenyl)-5-methylpyridazine;
* 3-(2-piperidino-2-methylpropyl)amino-6-phenyl-5-propylpyridazine;
* 3-(2-diethylamino-2-methylpropyl)amino-6-(4-chlorophenyl)-5-methylpyridazine;
* 3-(2-diethylamino-2-methylpropyl)amino-6-phenyl-5-methylpyridazine;
* 3-(2-diethylamino-2-methylpropyl)amino-5,6-diphenylpyridazine;
* 3-(2-diethylamino-2-methylpropyl)amino-6-(4-chlorophenyl)-5-propylpyridazine;
* 3-(2-diethylamino-2-methylpropyl)amino-6-(4-methoxyphenyl)-5-propylpyridazine;
* 3-(2-diethylamino-2-methylpropyl)amino-6-(4-hydroxyphenyl)-5-propylpyridazine;
* 3-(2-diethylamino-2-methylpropyl)amino-5-cyclopropyl-6-phenylpyridazine;
* 3-(2-diethylamino-2-methylpropyl)amino-5-isopropyl-6-(4-fluorophenyl)pyridazine;
* 3-(2-(morpholin-4-yl)-2-methylpropyl)amino-6-phenyl-5-propylpyridazine;
or a salt thereof with mineral or organic acids, characterized in that an amine of formula R₄NH₂ in which R₄ is a 2-diethylamino-2-methylpropyl or, if need be, 2-piperidino-2-methylpropyl or 2-(morpholin-4-yl)-2-methylpropyl group, is reacted with a 6-chloropyridazine of formula: 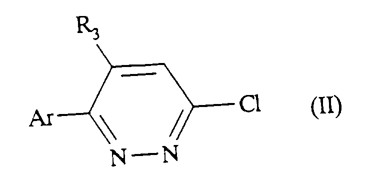
in which:
* R₃ is a propyl and Ar is a phenyl or, if need be, a 2-chlorophenyl, a 4-chlorophenyl, a 4-methoxyphenyl or a 4-hydroxyphenyl;
or
* R₃ is a methyl and Ar is a 2-methoxyphenyl or, if need be, a 2-hydroxyphenyl, a 4-chlorophenyl or a phenyl;
or
* R₃ is a phenyl and Ar is a phenyl;
or
* R₃ is a cyclopropyl and Ar a phenyl;
or
* R₃ is an isopropyl and Ar a 4-fluorophenyl;
and, optionally, the compound thus obtained is converted into a salt with a mineral or organic acid.

2. Process according to claim 1, for the preparation of the 3-(2-diethylamino-2-methylpropyl)amino-6-phenyl-5-propylpyridazine, characterized in that an amine R₄NH₂ in which R₄ is a 2-diethylamino-2-methylpropyl group is reacted with a 6-chloropyridazine (II) in which R₃ is a propyl and Ar is a phenyl.

3. Process according to claim 1, for the preparation of the 3-(2-diethylamino-2-methylpropyl)amino-5,6-diphenylpyridazine, characterized in that an amine R₄NH₂ in which R₄ is a 2-diethylamino-2-methylpropyl group is reacted with a 6-chloropyridazine (II) in which R₃ and Ar are each a phenyl.

4. Process for the preparation of a pharmaceutical composition containing as active principle a derivative obtained by the process of any one of claims 1 to 3, characterized in that it consists in mixing said derivative with at least one pharmaceutically acceptable excipient.

5. Process according to claim 4, characterized in that the active principle used is the derivative obtained according to the process of claim 2.

6. Process according to claim 4, characterized in that the active principle used is the derivative obtained according to the process of claim 3.

7. Process according to any one of claims 4 to 6, characterized in that the pharmaceutical composition contains from 0.1 to 100 mg of active principle per dosage unit.

## Claims (Claims for the following Contracting State(s): GR)

1. Pyridazine derivative, selected from:
* 3-(2-diethylamino-2-methylpropyl)amino-6-phenyl-5-propylpyrldazine;
* 3-(2-diethylamino-2-methylpropyl)amino-6-(2-chlorophenyl)-5-propylpyridazine;
* 3-(2-diethylamino-2-methylpropyl)amino-6-(2-methoxyphenyl)-5-methylpyridazine;
* 3-(2-diethylamino-2-methylpropyl)amino-6-(2-hydroxyphenyl)-5-methylpyridazine;
* 3-(2-piperidino-2-methylpropyl)amino-6-(4-chlorophenyl)-5-methylpyridazine;
* 3-(2-piperidino-2-methylpropyl)amino-6-phenyl-5-propylpyridazine;
* 3-(2-diethylamino-2-methylpropyl)amino-6-(4-chlorophenyl)-5-methylpyridazine;
* 3-(2-diethylamino-2-methylpropyl)amino-6-phenyl-5-methylpyridazine;
* 3-(2-diethylamino-2-methylpropyl)amino-5,6-diphenylpyridazine;
* 3-(2-diethylamino-2-methylpropyl)amino-6-(4-chlorophenyl)-5-propylpyridazine;
* 3-(2-diethylamino-2-methylpropyl)amino-6-(4-methoxyphenyl)-5-propylpyridazine;
* 3-(2-diethylamino-2-methylpropyl)amino-6-(4-hydroxyphenyl)-5-propylpyridazine;
* 3-(2-diethylamino-2-methylpropyl)amino-5-cyclopropyl-6-phenylpyridazine;
* 3-(2-diethylamino-2-methylpropyl)amino-5-isopropyl-6-(4-fluorophenyl)pyridazine;
* 3-(2-(morpholin-4-yl)-2-methylpropyl)amino-6-phenyl-5-propylpyridazine;
and the salts thereof with mineral or organic acids.

2. Derivative according to claim 1, which is the 3-(2-diethylamino-2-methylpropyl)amino-6-phenyl-5-propylpyridazine or a salt thereof with mineral or organic acids.

3. Derivative according to claim 1, which is the 3-(2-diethylamino-2-methylpropyl)amino-5,6-diphenylpyridazine or a salt thereof with mineral or organic acids.

4. Process for the preparation of the derivatives according to claim 1, characterized in that an amine of formula R₄NH₂ in which R₄ is a 2-diethylamino-2-methylpropyl or, if need be, 2-piperidino-2-methylpropyl or 2-(morpholin-4-yl)-2-methylpropyl group, is reacted with a 6-chloropyridazine of formula: 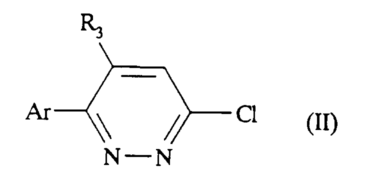 in which:
* R₃ is a propyl and Ar is a phenyl or, if need be, a 2-chlorophenyl, a 4-chlorophenyl, a 4-methoxyphenyl or a 4-hydroxyphenyl;
or
* R₃ is a methyl and Ar is a 2-methoxyphenyl or, if need be, a 2-hydroxyphenyl, a 4-chlorophenyl or a phenyl;
or
* R₃ is a phenyl and Ar is a phenyl;
or
* R₃ is a cyclopropyl and Ar a phenyl;
or
* R₃ is an isopropyl and Ar a 4-fluorophenyl;
and, optionally, the compound thus obtained is converted into a salt with a mineral or organic acid.

5. Process for the preparation of a pharmaceutical composition containing as active principle a derivative according to any one of claims 1 to 3, characterized in that it consists in mixing said derivative with at least one pharmaceutically acceptable excipient.

6. Process according to claim 5, characterized in that the active principle used is the derivative according to claim 2.

7. Process according to claim 5, characterized in that the active principle used is the derivative according to claim 3.

8. Process according to any one of claims 5 to 7, characterized in that the pharmaceutical composition contains from 0.1 to 100 mg of active principle per dosage unit.

## Patentansprüche (Patentansprüche für folgende(n) Vertragsstaat(en): AT, BE, CH, LI, DE, DK, FR, GB, IT, LU, NL, SE)

1. Pyridazin-Derivat, ausgewählt aus:
3-(2-Diethylamino-2-methylpropyl)-amino-6-phenyl-5-propylpyridazin;
3-(2-Diethylamino-2-methylpropyl)-amino-6-(2-chlorphenyl)-5-propylpyridazin;
3-(2-Diethylamino-2-methylpropyl)-amino-6-(2-methoxyphenyl)-5-methylpyridazin;
3-(2-Diethylamino-2-methylpropyl)-amino-6-(2-hydroxyphenyl)-5-methylpyridazin;
3-(2-Piperidino-2-methylpropyl)-amino-6-(4-chlorphenyl)-5-methylpyridazin;
3-(2-Piperidino-2-methylpropyl)-amino-6-phenyl-5-propylpyridazin;
3-(2-Diethylamino-2-methylpropyl)-amino-6-(4-chlorphenyl)-5-methylpyridazin;
3-(2-Diethylamino-2-methylpropyl)-amino-6-phenyl-5-methylpyridazin;
3-(2-Diethylamino-2-methylpropyl)-amino-5,6-diphenylpyridazin;
3-(2-Diethylamino-2-methylpropyl)-amino-6-(4-chlorphenyl)-5-propylpyridazin;
3-(2-Diethylamino-2-methylpropyl)-amino-6-(4-methoxyphenyl)-5-propylpyridazin;
3-(2-Diethylamino-2-methylpropyl)-amino-6-(4-hydroxyphenyl)-5-propylpyridazin;
3-(2-Diethylamino-2-methylpropyl)-amino-5-cyclopropyl-6-phenylpyridazin;
3-(2-Diethylamino-2-methylpropyl)-amino-5-isopropyl-6-(4-fluorphenyl)-pyridazin;
3-(2-(Morpholin-4-yl)-2-methylpropyl)-amino-6-phenyl-5-propylpyridazin;
und ihren Salzen mit Mineral- oder organischen Säuren.

2. Derivat nach Anspruch 1, welches 3-(2-Diethylamino-2-methylpropyl)-amino-6-phenyl-5-propylpyridazin oder eines seiner Salze mit Mineral- oder organischen Säuren ist.

3. Derivat nach Anspruch 1, welches 3-(2-Diethylamino-2-methylpropyl)-amino-5,6-diphenylpyridazin oder eines seiner Salze mit Mineral- oder organischen Säuren ist.

4. Verfahren zur Herstellung von Derivaten nach Anspruch 1, dadurch gekennzeichnet, daß ein Amin der Formel R₄NH₂, worin R₄ eine Gruppe 2-Diethylamino-2-methylpropyl oder gegebenenfalls 2-Piperidino-2-methylpropyl oder 2-(Morpholin-4-yl)-2-methylpropyl bedeutet, umgesetzt wird mit einem 6-Chlor pyridazin der Formel: 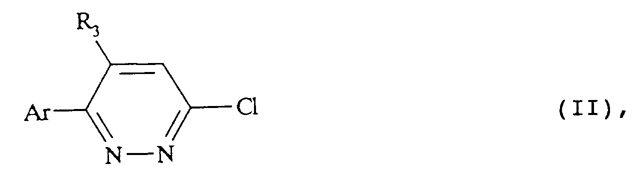 worin R₃ Propyl bedeutet, und Ar Phenyl oder gegebenenfalls 2-Chlorphenyl, 4-Chlorphenyl, 4-Methoxyphenyl oder 4-Hydroxyphenyl darstellt; oder R₃ Methyl bedeutet, und Ar 2-Methoxyphenyl oder gegebenenfalls 2-Hydroxyphenyl, 4-Chlorphenyl oder Phenyl darstellt; oder R₃ Phenyl bedeutet, und Ar Phenyl darstellt; oder R₃ Cyclopropyl und Ar Phenyl bedeutet; oder R₃ Isopropyl und Ar 4-Fluorphenyl darstellt; und gegebenenfalls die so erhaltene Verbindung mit einer Mineral- oder organischen Säure versalzt wird.

5. Pharmazeutische Zusammensetzung, dadurch gekennzeichnet, daß sie als aktiven Bestandteil ein Derivat nach einem der Ansprüche 1 bis 3 enthält.

6. Pharmazeutische Zusammensetzung nach Anspruch 5, dadurch gekennzeichnet, daß sie als aktiven Bestandteil ein Derivat nach Anspruch 2 enthält.

7. Pharmazeutische Zusammensetzung nach Anspruch 5, dadurch gekennzeichnet, daß sie als aktiven Bestandteil ein Derivat nach Anspruch 3 enthält.

8. Pharmazeutische Zusammensetzung nach einem der Ansprüche 5 bis 7, dadurch gekennzeichnet, daß sie 0,1 bis 100 mg aktiven Bestandteil pro Dosierungseinheit umfaßt.

## Patentansprüche (Patentansprüche für folgende(n) Vertragsstaat(en): ES)

1. Verfahren zur Herstellung eines Pyridazin-Derivats, ausgewählt aus:
3-(2-Diethylamino-2-methylpropyl)-amino-6-phenyl-5-propylpyridazin;
3-(2-Diethylamino-2-methylpropyl)-amino-6-(2-chlorphenyl)-5-propylpyridazin;
3-(2-Diethylamino-2-methylpropyl)-amino-6-(2-methoxyphenyl)-5-methylpyridazin;
3-(2-Diethylamino-2-methylpropyl)-amino-6-(2-hydroxyphenyl)-5-methylpyridazin;
3-(2-Piperidino-2-methylpropyl)-amino-6-(4-chlorphenyl)-5-methylpyridazin;
3-(2-Piperidino-2-methylpropyl)-amino-6-phenyl-5-propylpyridazin;
3-(2-Diethylamino-2-methylpropyl)-amino-6-(4-chlorphenyl)-5-methylpyridazin;
3-(2-Diethylamino-2-methylpropyl)-amino-6-phenyl-5-methylpyridazin;
3-(2-Diethylamino-2-methylpropyl)-amino-5,6-diphenylpyridazin;
3-(2-Diethylamino-2-methylpropyl)-amino-6-(4-chlorphenyl)-5-propylpyridazin;
3-(2-Diethylamino-2-methylpropyl)-amino-6-(4-methoxyphenyl)-5-propylpyridazin;
3-(2-Diethylamino-2-methylpropyl)-amino-6-(4-hydroxyphenyl)-5-propylpyridazin;
3-(2-Diethylamino-2-methylpropyl)-amino-5-cyclopropyl-6-phenylpyridazin;
3-(2-Diethylamino-2-methylpropyl)-amino-5-isopropyl-6-(4-fluorphenyl)-pyridazin;
3-(2-(Morpholin-4-yl)-2-methylpropyl)-amino-6-phenyl-5-propylpyridazin;
oder eines ihrer Salze mit Mineral- oder organischen Säuren, dadurch gekennzeichnet, daß ein Amin der Formel R₄NH₂, worin R₄ eine Gruppe 2-Diethylamino-2-methylpropyl oder gegebenenfalls 2-Piperidino-2-methylpropyl oder 2-(Morpholin-4-yl)-2-methylpropyl bedeutet, umgesetzt wird mit einem 6-Chlor pyridazin der Formel: 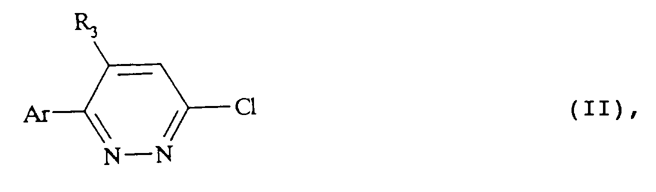 worin R₃ Propyl bedeutet, und Ar Phenyl oder gegebenenfalls 2-Chlorphenyl, 4-Chlorphenyl, 4-Methoxyphenyl oder 4-Hydroxyphenyl darstellt; oder R₃ Methyl bedeutet, und Ar 2-Methoxyphenyl oder gegebenenfalls 2-Hydroxyphenyl, 4-Chlorphenyl oder Phenyl darstellt; oder R₃ Phenyl bedeutet, und Ar Phenyl darstellt; oder R₃ Cyclopropyl und Ar Phenyl bedeutet; oder R₃ Isopropyl und Ar 4-Fluorphenyl darstellt; und gegebenenfalls die so erhaltene Verbindung mit einer Mineral- oder organischen Säure versalzt wird.

2. Verfahren nach Anspruch 1 zur Herstellung von 3-(2-Diethylamino-2-methylpropyl)-amino-6-phenyl-5-propylpyridazin, dadurch gekennzeichnet, daß ein Amin der Formel R₄NH₂, worin R₄ eine Gruppe 2-Diethylamino-2-methylpropyl bedeutet, umgesetzt wird mit einem 6-Chlor pyridazin (II), worin R₃ Propyl und Ar Phenyl bedeutet.

3. Verfahren nach Anspruch 1 zur Herstellung von 3-(2-Diethylamino-2-methylpropyl)-amino-5,6-diphenylpyridazin, dadurch gekennzeichnet, daß ein Amin der Formel R₄NH₂, worin R₄ eine Gruppe 2-Diethylamino-2-methylpropyl bedeutet, umgesetzt wird mit einem 6-Chlor pyridazin (II), worin R₃ und Ar jeweils Phenyl bedeuten.

4. Verfahren zur Herstellung einer pharmazeutischen Zusammensetzung, die als aktiven Bestandteil ein durch das Verfahren nach einem der Ansprüche 1 bis 3 erhaltenes Derivat enthält, dadurch gekennzeichnet, daß es aus dem Mischen des Derivats mit zumindest einem pharmazeutisch annehmbaren Exzipienten besteht.

5. Verfahren nach Anspruch 4, dadurch gekennzeichnet, daß als aktiver Bestandteil das gemäß dem Verfahren nach Anspruch 2 erhaltene Derivat verwendet wird.

6. Verfahren nach Anspruch 4, dadurch gekennzeichnet, daß als aktiver Bestandteil das gemäß dem Verfahren nach Anspruch 3 erhaltene Derivat verwendet wird.

7. Verfahren nach einem der Ansprüche 4 bis 6, dadurch gekennzeichnet, daß die pharmazeutische Zusammensetzung 0,1 bis 100 mg aktiven Bestandteil pro Dosierungseinheit enthält.

## Patentansprüche (Patentansprüche für folgende(n) Vertragsstaat(en): GR)

1. Pyridazin-Derivat, ausgewählt aus:
3-(2-Diethylamino-2-methylpropyl)amino-6-phenyl-5-propylpyridazin;
3-(2-Diethylamino-2-methylpropyl)-amino-6-(2-chlorphenyl)-5-propylpyridazin;
3-(2-Diethylamino-2-methylpropyl)-amino-6-(2-methoxyphenyl)-5-methylpyridazin;
3-(2-Diethylamino-2-methylpropyl)-amino-6-(2-hydroxyphenyl)-5-methylpyridazin;
3-(2-Piperidino-2-methylpropyl)-amino-6-(4-chlorphenyl)-5-methylpyridazin;
3-(2-piperidino-2-methylpropyl)-amino-6-phenyl-5-propylpyridazin;
3-(2-Diethylamino-2-methylpropyl)-amino-6-(4-chlorphenyl)-5-methylpyridazin;
3-(2-Diethylamino-2-methylpropyl)-amino-6-phenyl-5-methylpyridazin;
3-(2-Diethylamino-2-methylpropyl)-amino-5,6-diphenylpyridazin;
3-(2-Diethylamino-2-methylpropyl)-amino-6-(4-chlorphenyl)-5-propylpyridazin;
3-(2-Diethylamino-2-methylpropyl)-amino-6-(4-methoxyphenyl)-5-propylpyridazin;
3-(2-Diethylamino-2-methylpropyl)-amino-6-(4-hydroxyphenyl)-5-propylpyridazin;
3-(2-Diethylamino-2-methylpropyl)-amino-5-cyclopropyl-6-phenylpyridazin;
3-(2-Diethylamino-2-methylpropyl)-amino-5-isopropyl-6-(4-fluorphenyl)-pyridazin;
3-(2-(Morpholin-4-yl)-2-methylpropyl)-amino-6-phenyl-5-propylpyridazin;
und ihren Salzen mit Mineral- oder organischen Säuren.

2. Derivat nach Anspruch 1, welches 3-(2-Diethylamino-2-methylpropyl)-amino-6-phenyl-5-propylpyridazin oder eines seiner Salze mit Mineral- oder organischen Säuren ist.

3. Derivat nach Anspruch 1, welches 3-(2-Diethylamino-2-methylpropyl)-amino-5,6-diphenylpyridazin oder eines seiner Salze mit Mineral- oder organischen Säuren ist.

4. Verfahren zur Herstellung von Derivaten nach Anspruch 1, dadurch gekennzeichnet, daß ein Amin der Formel R₄NH₂, worin R₄ eine Gruppe 2-Diethylamino-2-methylpropyl oder gegebenenfalls 2-Piperidino-2-methylpropyl oder 2-(Morpholin-4-yl)-2-methylpropyl bedeutet, umgesetzt wird mit einem 6-Chlor pyridazin der Formel: 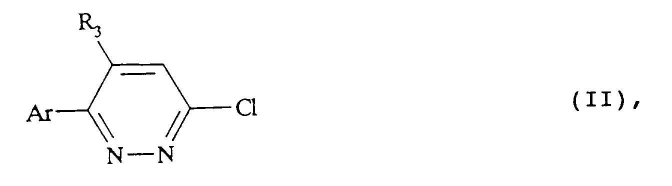 worin R₃ Propyl bedeutet, und Ar Phenyl oder gegebenenfalls 2-Chlorphenyl, 4-Chlorphenyl, 4-Methoxyphenyl oder 4-Hydroxyphenyl darstellt; oder R₃ Methyl bedeutet, und Ar 2-Methoxyphenyl oder gegebenenfalls 2-Hydroxyphenyl, 4-Chlorphenyl oder Phenyl darstellt; oder R₃ Phenyl bedeutet, und Ar Phenyl darstellt; oder R₃ Cyclopropyl und Ar Phenyl bedeutet; oder R₃ Isopropyl und Ar 4-Fluorphenyl darstellt; und gegebenenfalls die so erhaltene Verbindung mit einer Mineral- oder organischen Säure versalzt wird.

5. Verfahren zur Herstellung einer pharmazeutischen Zusammensetzung, die als aktiven Bestandteil ein Derivat nach einem der Ansprüche 1 bis 3 enthält, dadurch gekennzeichnet, daß es aus dem Mischen des Derivats mit zumindest einem pharmazeutisch annehmbaren Exzipienten besteht.

6. Verfahren nach Anspruch 5, dadurch gekennzeichnet, daß als aktiver Bestandteil das Derivat nach Anspruch 2 verwendet wird.

7. Verfahren nach Anspruch 5, dadurch gekennzeichnet, daß als aktiver Bestandteil das Derivat nach Anspruch 3 verwendet wird.

8. Verfahren nach einem der Ansprüche 5 bis 7, dadurch gekennzeichnet, daß die pharmazeutische Zusammensetzung 0,1 bis 100 mg aktiven Bestandteil pro Dosierungseinheit enthält.
